# EUROPEAN PATENT APPLICATION

(11) **EP 4 067 729 A1**
(43) Date of publication of application: **05.10.2022**
(21) Application number: 21460017.3
(22) Date of filing: 31.03.2021
(51) Int. Cl.: F21S 6/00, F21S 4/28, F21S 8/00, F21Y 115/10, F21Y 113/10, F21Y 113/20, F21Y 103/00

(54) **SOLAR LIGHT SIMULATOR LAMP**

(71) Applicant: TISOFT, Wojciech Jedrzejewski, 88-100 Inowroclaw (PL)
(72) Inventor: Wojciech, Jedrzejewski, 88-100 Inowroclaw (PL)
(74) Representative: Rozga, Blazej

(57) **Abstract**

The invention relates to the lamp that replenishes light deficiencies for everyday use. Lamps, with additional accessories, e.g. stand, wall mount, ceiling mount can be used in households, offices, cafes, restaurants, home saunas, bathrooms, break rooms, therapy offices. A solar light simulator lamp for emitting a mixture of light of different wavelengths, as the radiation source includes UV radiation lamps, visible light lamps and infrared lamps, with adjustable power for each source, electronically controlled.

## Description

The invention relates to the lamp that replenishes light deficiencies for everyday use. Lamps, with additional accessories, e.g. stand, wall mount, ceiling mount can be used in households, offices, cafes, restaurants, home saunas, bathrooms, break rooms, therapy offices and other places where people stay. The invention serves as a device for everyday light therapy aimed to compensate for the deficiency in sunlight. The device has a particularly favourable effect on areas with little sunlight.

An example of an electric infrared heater is known from the patent description No. PL 210605 B1. It is a device designed, among others for heating people, for providing energy to the body, in particular during treatment with heat. Such treatment is based on thermal radiation.

Solariums are known to be widely used. In these devices, radiators generate UV light for tanning. Such equipment consists of a cabin in which fluorescent lamps shining in the UV spectrum are installed. These devices are known as tanning beds or tanning stand-up beds. Use of the solarium should not exceed a safe, relatively short period of time.

The device described in the Polish translation of the patent description No. PL / EP 2663802 is also known, in which the invention relates to a solar light simulator comprising high-intensity discharge lamps type as well as halogen lamps type, which are applied simultaneously and provided with infrared filter. The device generates radiation similar approximating radiated sunlight.
It is medically proven that UV-B radiation activates the body to produce vitamin D3, which allows proper functioning of the body. This knowledge was described in the article by E. Skórska "The impact of solar ultraviolet radiation on the human body", 2016. There are regions where humans are exposed to sunlight deficiencies. An example is Poland, which was described in the article by J. Biszczuk-Jakubowska, A. Curylo "Solar UV radiation and human health", 2010. It is known that sunlight improves the comfort of staying indoors. The device according to the invention reduces the sunlight deficiency in humans.

It is known from the prior art, e.g. in US 5623149, that many types of lamps can be applied as solar light sources in solar simulators. According to the applicants, it is preferred that a plurality of discharge lamps and a plurality of halogen lamps are applied. This arrangement promotes adequate mixing of light of both types of lamps and provides an appropriate light spectrum.

The essence of the solution of a solar light simulator lamp to emit a mixture of light with different wavelengths, is the fact that the sources of UV radiation are the UV radiation discharge lamps, LED visible light lamps as well as infrared lamps mounted on a movable bar, electronically controlled.

In the solution, three sources of radiation were applied, which makes the lamp spectrum more similar to the spectrum of sunlight, which was not the subject of previous solutions.

The lamp module according to the invention consists of a housing to which a discharge lamp as a UV light source, tapes containing LED diodes as a visible light source and halogen filaments as infrared light sources were attached. Halogen filaments generate visible light additionally. The power of LED strips and halogen lamps is controlled by pulse width modulation (PWM) by means of two separate modules inside the driver. The power control of UV discharge lamps is carried out with a variable supply frequency on the electronic ballast which is a part of the driver. Both PWM modules and the electronic ballast are controlled by a microprocessor. The device's power supply is integrated with the driver. The device is powered by the electrical grid of 230V AC.

The discharge lamp has a power raging from 75 W to 200 W and generates radiation in the range of UVA (wavelength range from 315 nm to 400 nm) with an intensity of 32 W / m² and UVB (wavelength range from 280 nm to 315 nm) with an intensity of about 840 mW / m².

The LED strip has a power raging from 1 W to 30 W and generates visible light with a wavelength range from 550 nm to 660 nm.

A single halogen lamp has a power raging from 30 W to 200 W and generates light in the wavelength range from 560 nm to 1000 nm.

It is advantageous to apply an electronic driver that controls the power of the light sources depending on the operating mode selected by the user. The device may work in heating, lighting as well as solarium mode, adjusting the light spectrum to the spectrum of sunlight.

Generating light in the infrared, visible and ultraviolet range in variable proportions, allows applying the lamp for various purposes, e.g. for rooms lighting, sunbathing, rooms heating, improving the comfort of staying in a given place, light treatment as well as heating up parts of the body or growing plants.

It is advantageous to apply a lamp module for which various configurations of additional equipment are selected. Such a construction makes allows producing of many versions of the device that are adapted to different applications.

In the device designed for the utility room, office or break room a stand is applied. The stand is equipped with swivel wheels, to which a lamp module with light sources is vertically mounted. The swivel wheels make the device mobile. The vertical orientation of the lamp module makes the device compact and space-saving.

The device designed for permanent mounting to the wall in a shower cabin, sauna or swimming pool is equipped with a wall holder to which a lamp module with light sources and a driver is attached.. The device is additionally equipped with a bolted housing with rubber gaskets ensuring moisture resistance with IP65 protection degree.

The device is designed to be mounted on the ceiling in the form of a ceiling lamp. It consists of a lamp module containing light sources and a controller as well as a holder for mounting the object under the ceiling. The device replaces traditional ceiling lamps without taking up additional space in the room.

The advantage of the subject of the invention is summing up the advantages of the component solution as well as the ability to adjust the operation the lamp/simulator. Several applications of the invention are possible, e. g. as a heating lamp, for safe and natural tanning, lighting rooms, improving comfort or in healing. The advantage of the invention is the possibility of application the positive effects of light composed of sunlight. The use of the device in regions with low sunlight is especially beneficial. The solution will work in locations where the user spends most of the day at work and cannot use natural sunlight. The use of the invention by people who are in isolation, e. g. due to health problems or limited mobility, has positive effects.

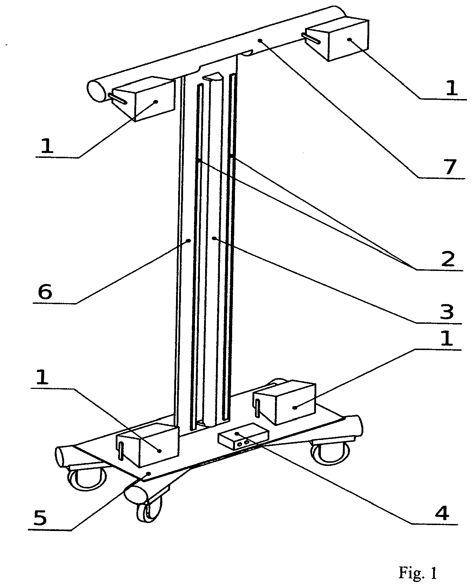

The invention relating to a solar light simulator lamp has been shown in an embodiment. The subject of the invention has been illustrated in the drawing in which Fig.1 shows a front view of the solar radiation simulator lamp.

### Example 1

The subject of the invention in the embodiment is shown in the drawing 1, which shows the location of the IR source, visible light source in the form of a strip with LED diodes 2 and a discharge lamp as a UV light source 3. Light sources 1, 2 and 3 are electrically connected to the electronic controller 4. Lower halogen lamps 1 are attached to the base of the housing with swivel wheels 5, to which the housing body 6 is also mounted. The discharge lamp 3 and the LED strips 2 are mounted to the housing body. The upper frame 7 for mounting the upper halogens 1 is attached to the upper part of the body.

### Example 2

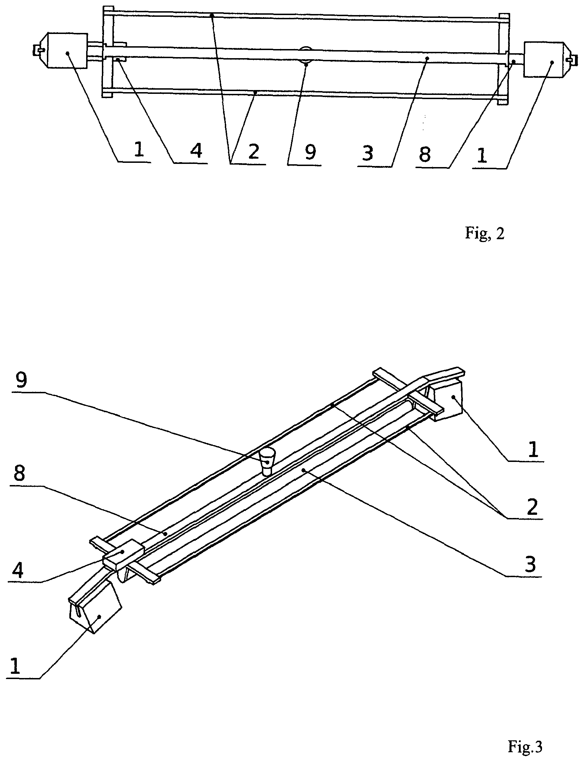

The subject matter of the invention in an example of a ceiling lamp is illustrated in Fig. 2 and Fig. 3. It is constructed from halogen lamps 1 which are attached to the lamp body 8. The body is connected to a discharge lamp 3. The LED strips 2 are fixed by brackets 8 to the lamp body. The LED strips 2, the discharge lamp 3 and the halogen lamps 1 are electrically connected to the electronic controller 4, which is mounted to the lamp body 8. The lamp body is connected with the bracket for mounting the device to the ceiling 9. The cable that powers the integrated power supply in the controller 4 is routed through the bracket.

Various modifications and different varieties of the described methods and system according to the invention will be apparent to those skilled in the art without departing from the scope of the invention.

Although the invention has been described in connection with certain preferred embodiments, it should be understood that the invention, as it has been claimed, should not be limited to such specific embodiments and that many modifications and additions may be performed within the scope of the present solutions.

## Claims

1. A solar light simulator lamp to emit a mixture of light with different wavelengths, distinguished by that it comprises UV radiation lamps, visible light lamps and infrared radiators as the radiation source, with adjustable power for each source, electronically controlled.

2. The lamp according to the claim 1, distinguished by that the UV radiation discharge lamp generates UVA radiation ranging from 315 nm to 400 nm with an intensity of 32 W/m² as well as UVB radiation ranging from 280nm to 400 nm with an intensity of 840 m W/m².

3. The lamp according to the claim 1, distinguished by that the visible light with the wavelength ranging from 550 nm to 660 nm is generated by LED diodes in the form of strips.

4. The lamp according to the claim 1, distinguished by that halogen filaments are the infrared emitters generating radiation ranging from 560 nm to 1000 nm.

5. The lamp according to the claims 2, 3 and 4, distinguished by that the power, range of operation of the individual lamp is electronically controlled by the microprocessor control unit.

6. The control unit according to claim 5, distinguished by that the power of LED strips and IR halogen lamps is controlled by pulse width modulation - PWM by means of two modules.

7. The control unit according to claim 5, distinguished by that the control of the UV discharge lamps is carried out with a variable power frequency in the microprocessor control unit.

8. The control unit according to the claim 5, distinguished by that LED lamps and IR radiation filaments are controlled so that their power and radiation range are consistent with the power and wavelength range of UV discharge lamps.

9. The lamp solar light simulator, distinguished by that the lamps according to the claims 2, 3 and 4 emitting individual light and radiation are integrated in the form of a bar that may be mounted on a movable stand, in a shower cabin or may be hung on the wall of any room.
